(19) Europäisches Patentamt
European Patent Office
Office européen des brevets

(11) Publication number: **0 419 890 A2**

# (12) EUROPEAN PATENT APPLICATION

(21) Application number: **90116842.7**

(22) Date of filing: **03.09.90**

(51) Int. Cl.[5]: **A61K 31/675**, A61K 31/53, A61K 47/10

(30) Priority: **29.09.89 US 414500**

(43) Date of publication of application: **03.04.91 Bulletin 91/14**

(84) Designated Contracting States: **AT BE CH DE DK ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Ritter, Lawrence**
**25 Hillcrest Road**
**Suffern, New York 10901(US)**
Inventor: **Haeger, Bruce Edwin**
**15 Corona Road**
**Highland Mills, New York 10930(US)**

(74) Representative: **Wächtershäuser, Günter, Dr.**
**Tal 29**
**W-8000 München 2(DE)**

## (54) Stable pharmaceutical formulations for antineoplastic compounds having more than one ethyleneimine group, and method.

(57) Preconstituted pharmaceutical compositions comprising antineoplastic compounds having more than one ethyleneimine group are provided by adding a water-free, water-miscible, neutral or basic, oxygen containing pharmaceutically acceptable solvent. Such compositions remain stable for prolonged periods under refrigerated conditions and are reconstitutable in water or other I.V. diluents to provide injectable dosage forms.

EP 0 419 890 A2

# STABLE PHARMACEUTICAL FORMULATIONS FOR ANTINEOPLASTIC COMPOUNDS HAVING MORE THAN ONE ETHYLENEIMINE GROUP, AND METHOD

The present invention relates to new improved compositions comprising antineoplastic compounds having more than one ethyleneimine group, and a solvent. More particularly, it relates to compositions comprising, e.g., triethylenemelamine, triethylenephosphoramide, triethylenethiophosphoramide and the like, that remain stable for prolonged periods under refrigerated conditions because a water-free, water-miscible, neutral or basic, oxygen containing pharmaceutically acceptable solvent is utilized to stabilize the compositions.

## BACKGROUND OF THE INVENTION

Antineoplastic compounds having more than one ethyleneimine group are known to be pharmaceutically effective. They are polyfunctional alkylating agents used in the chemotherapy of certain neoplastic diseases. The alkylating groups react with nucleophilic centers in many different kinds of molecules, and their bi- or trifunctional character allows them to cross-link doublestranded DNA, thus preventing the strands from separating for replication. See Remington's Pharmaceutical Sciences, Seventeenth Edition, Mack Publishing Co., Easton, PA 1985 (Remington's 17th Ed.) p. 1141.

Triethylenemelamine is an antineoplastic compound having the formula:

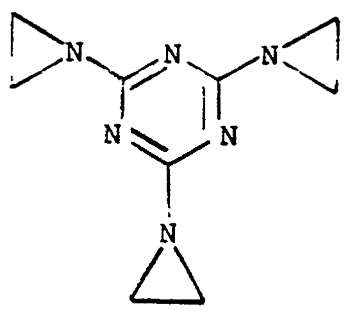

Merck Index, Tenth Edition (Merck 10th Ed.), p.9478. Wystrach, Kaiser, U.S. 2,520,619, describe a method of preparing triethylenemelamine by simple and efficient means, and the recovery of the triethylenemelamine so formed. In addition to its pharmaceutical use, it is also used in the manufacture of resinous products, textile finishing agents, and as an insect sterilant. Merck 10th Ed., p. 9478.

Kaiser, Schaefer, U.S. 2,653,934 claim mono-and bis-ethylenimino-s-triazines of the formula:

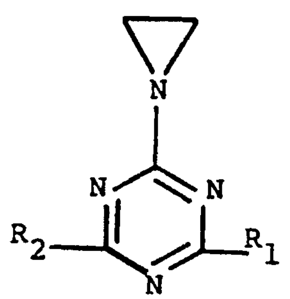

in which $R_1$ is a member of the group consisting of hydrogen, halogen, alkoxy, aromatic hydrocarbon, and amino, and $R_2$ is a member of the group consisting of $R_1$ and ethylenimino. Compounds of the above class are useful in pharmaceutical preparations. The compounds are also useful in resins, and in textile treatment.

Triethylenephosphoramide is also an antineoplastic compound. It has the following formula:

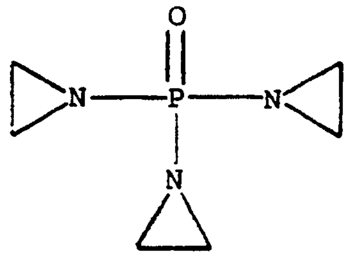

It is also used as an insect chemosterilant, in dyeing, creaseproofing and flameproofing textiles, as a stabilizer for polymers, and in photographic emulsion hardening. Merck 10th Ed., p. 9478.

Triethylenethiophosphoramide is an antineoplastic compound of the following formula:

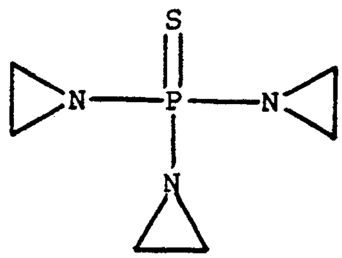

The compound was first disclosed in Kuh, Seeger, U.S. 2,670,347, which included a claim for the compound and a claim for a method of preparing triethylenethiophosphoramide which comprises reacting thiophosphoryl chloride with ethylenimine in a solvent and recovering said compound therefrom. EPO Patent Publication No. 894,820, describes a method of obtaining triethylenethiophosphoramide, in a state of high purity and free of resinuous products. In addition to its pharmaceutical use, it is also used as an insect sterilant. Merck 10th Ed., p. 9479.

Triethylenethiophosphoramide is available commercially, sold under the name of Thiotepa as a parenteral product. It is available in powder form, in vials which contain a sterile mixture of 15 mg triethylenethiophosphoramide, 80 mg sodium chloride and 50 mg sodium bicarbonate per vial. Physician's Desk Reference, Forty-third Edition, Medical Economics Company, Oradell, NJ 1989 (PDR 43rd Ed.) p. 1152. Parenteral routes of administration are most reliable since absorption of Thiotepa from the gastrointestinal tract is variable. It may be given by rapid intravenous administration, injected directly into a tumor by means of a 22-gauge needle, instilled into cavities through the same tubing which is used to remove the fluid from the cavity involved, or administered intravesically by catheter. See PDR 43rd Ed., p. 1152. See also Remington's 17th Ed., pp. 1156-1157.

Thiotepa is presently used in the chemotherapy of certain neoplastic diseases in a solution form reconstituted with water. The preparation of the solution form normally involves two steps. Firstly, the powder should be reconstituted preferably in Sterile Water for Injection. The amount of diluent most often used is 1.5 ml resulting in a drug concentration of 5 mg in each 0.5 ml of solution. Larger volumes are usually employed for intracavitary use, intravenous drip, or perfusion therapy. The solution should be mixed well and its opaqueness observed. Reconstituted solutions should be clear to slightly opaque but solutions that are grossly opaque or precipitated should not be used. Secondly, the reconstituted solution is often added to larger volumes of other diluents: Sodium Chloride Injection USP, Dextrose Injection USP, Dextrose and Sodium Chloride Injection USP, Ringer's Injection USP, or Lactated Ringer's Injection USP. During both steps of reconstitution, procedures for proper handling and disposal of anti-cancer drugs should be considered. See PDR 43rd Ed., p. 1152.

Whether in its original powder form or in the solution form reconstituted with water, Thiotepa must be stored in a refrigerator at 2-8 °C. However, in its solution form reconstituted with water, Thiotepa can be stored for only 5 days in a refrigerator without substantial loss of potency. See PDR 43rd Ed., p. 1152. Thus, the product must be reconstituted with water at the time of usage, or at a short time before usage.

Similarly, ampuled aqueous solutions of triethylenemelamine are stable for only about 3 months when stored in a refrigerator at 4 °C. Merck 10th Ed., p. 9478.

It has now been found that the loss of potency experienced with refrigerated solutions of powder

reconstituted with water can be overcome in accordance with this invention by preconstituted dosage forms prepared with a water-free, water-miscible, neutral or basic, oxygen-containing pharmaceutically acceptable solvent.

Surprisingly, the new composition of a preconstituted solution, made up of compounds having more than one ethyleneimine group and water-free, water-miscible, neutral or basic, oxygen-containing pharmaceutically acceptable solvents, has been found to be superior to the presently used solution form reconstituted with water, in terms of ability to maintain potency under refrigerated conditions. These new compositions make it possible to store the preconstituted solution for a minimum of one year under refrigerated conditions (2° to 8° C), in contrast to the 5 day shelf-life of the reconstituted Thiotepa formula, or the 3 month shelf-life of the reconstituted triethylenemelamine formula. These new compositions further make it possible to provide the potential for development of a broader dosage line for products such as, e.g. single or multi-dose preconstituted vials containing greater volumes and varied concentrations of the compounds having more than one ethyleneimine group. These new compositions also make it possible to provide the potential for development of a dosage form reconstitutable with pharmaceutically acceptable additives, such as procaine HCl or epinephrine HCl.

Unexpectedly also, the first step of the two normal reconstitution steps described above will usually be eliminated. The preconstituted solution cannot be used directly for parenteral use, but where it is to be added to larger volumes of other diluents, the initial reconstitution step is avoided. A further unexpected result is that the preconstituted formulation is self preserving, thus guaranteeing the sterility of the product at the time of use.

## SUMMARY OF THE INVENTION

According to the invention there are provided stable injectable pharmaceutical compositions, reconstituted with water from a solution comprising (i) an effective amount of an antineoplastic compound having more than one ethyleneimine group; and (ii) a water-free, water-miscible, neutral or basic, oxygen containing pharmaceutically acceptable solvent therefor. In preferred embodiments, the compositions comprise those wherein said antineoplastic compound comprises triethylenethiophosphoramide, triethylenemelamine, triethylenephosphoramide or a mixture of any of the foregoing; those wherein said antineoplastic compound comprises triethylenethiophosphoramide; those wherein said solvent is selected from dehydrated alcohol, glycerine, a polyethylene glycol, propylene glycol, or a mixture of any of the foregoing. Especially preferred are compositions wherein said solvent comprises a polyethylene glycol; those wherein said solvent comprises polyethylene glycol 300; those wherein said solvent comprises dehydrated alcohol. Special mention is made of a preconstituted pharmaceutical solution, adapted to form an injectable aqueous composition comprising (i) an effective amount of an antineoplastic compound having more than one ethyleneimine group; and (ii) a polyethylene glycol; those wherein said antineoplastic compound comprises triethylenethiophosphoramide, triethylenemelamine, triethylenephosphoramide or a mixture of any of the foregoing; those wherein said antineoplastic compound comprises triethylenethiophosphoramide; those wherein said solvent comprises polyethylene glycol 300; those wherein said solution comprises from 5 mg/ml to 30 mg/ml of triethylenethiophosphoramide in polyethylene glycol 300; and particularly one in which the concentration of triethylenethiophosphoramide is from 9.5 mg/ml to 11.0 mg/ml in polyethylene glycol 300.

The present invention also contemplates a method for producing stable injectable pharmaceutical compositions comprising the steps of (A) providing (i) an effective amount of an antineoplastic compound having more than one ethyleneimine group; (B) adding thereto (ii) a water-free, water-miscible, neutral or basic, oxygen containing pharmaceutically acceptable solvent; and (C) reconstituting the product of step B in water.

## DETAILED DESCRIPTION OF THE INVENTION

The composition of this invention can be prepared by techniques well known to those skilled in the art of pharmaceutical formulation. The substantially pure antineoplastic compounds having more than one ethylene group may be prepared, mixed with the other components, filled into clean containers, the containers sealed with teflon coated butyl closures, and stored in a refrigerator at 2-8° C.

Triethylenemelamine can be prepared by a method comprising admixing water, cyanuric chloride, ethyleneimine, and a hydrogen chloride acceptor of the group consisting of the alkali metal hydroxides, alkali metal carbonates, alkaline earth carbonates, at a temperature below 20° C whereby hydrogen chloride and triethylenemelamine are formed, and recovering the latter, in accordance with U.S. 2,520,619.

The preparation of triethylenephosphoramide is described in Bestian, Ann . 566, 231 (1950).

Triethylenethiophosphoramide can be prepared by reacting N-metallo-derivative of ethyleneimine with a phosphorus thiohalide at a temperature between -30 and +15° C in an inert anhydrous organic solvent, in accordance with EPO Patent Application No. 894,820. It is an item of commerce, and is available from Lederle Laboratories (a division of American Cyanamid Company, Wayne, New Jersey) under the name of Thiotepa (PDR 43rd Ed., p. 1152).

Dehydrated alcohol, glycerine, and propylene glycol are items of commerce and, if used, are widely available from a number of sources (Remington's 17th Ed., pp. 1309, 1308 and 1309 respectively).

Polyethylene glycols 200, 300, 400 and 600 are clear, viscous liquids at room temperature. They are items of commerce and, if used, are widely available from a number of sources (Remington's 17th Ed., p. 1305).

The amounts of the respective components can vary fairly broadly, within conventional limits well known to those skilled in the art. Preferred embodiments will be exemplified hereinafter. Typically the triethylenemelamine, triethylenephosphoramide or triethylenethiophosphoramide will comprise from about 1 to 60 mg/ml, preferably from about 2 to 45 mg/ml and especially preferably from about 5 to 30 mg/ml. Special mention is made of from 9.5 to 11.0 mg/ml of triethylenemelamine, triethylenephosphoramide or triethylenethiophosphoramide.

The amounts of the solvent or solvents utilized can vary broadly, e.g., from about 50 to 100 percent w/v, preferably more than 90 percent w/v, and especially preferably 100 percent w/v.

The solutions prepared as described above and more fully exemplified hereinafter are used in conventional dosages. A typical daily dose of the antineoplastic compounds having more than one ethyleneimine group depends on the type of application. A typical parenteral dose of Thiotepa is generally from 0.3 to 0.4 mg/kg at intervals of from 1 to 4 weeks. Typical intrapleural, intraperitoneal, intrapericardial or intratumor dosages are generally from 0.6 to 0.8 mg/kg once a week, but are discontinued if the leukocyte count is below 3000 cells/mm3, until the count rises above that value. For maintenance, dosages of from 0.07 to 0.8 mg/kg are given every 1 to 4 weeks. In malignant ascites, a typical intraperitoneal dose is from 10 to 15 mg once a week as long as leukocyte and platelet counts are sufficient.

## DESCRIPTION OF THE PREFERRED EMBODIMENTS

The following examples illustrate the invention but are not intended to limit the claims in any manner whatsoever.

### EXAMPLE 1

Triethylenethiophosphoramide is used as the active ingredient, and polyethylene glycol 300 is used as the solvent, in a composition having the formula set forth in Table 1:

TABLE 1.

| COMPOSITION FOR INJECTION COMPRISING TRIETHYLENETHIOPHOSPHORAMIDE | | |
|---|---|---|
| Ingredient | % w/v | Rationale |
| TRIETHYLENETHIOPHOSPHORAMIDE | 2.0 | active ingredient |
| 300 NF qs ad | 100.0 | vehicle |

The preparation of the formulation requires the handling of triethylenethiophosphoramide which is

considered to be a toxic material, so all personnel involved with the preparation are fully trained in its handling.

A 400 ml volume of polyethylene glycol 300 NF is transferred to a suitable beaker. 10.09 g of milled triethylenethiophosphoramide (99.1 percent) is added and dissolved with mild agitation. The solution is diluted to exactly 500 ml with polyethylene glycol 300 and mixed well. The above prepared parenteral solution is filled into two sets of Type 1, clear glass vials, labeled Group 1 and Group 2.

Group 1 consists of 100 vials, each receiving 3.17 ml of solution. These vials are plugged with butyl rubber closures and capped with red, 13 mm flip off caps.

Group 2 consists of 42 vials, each receiving 3.17 ml of solution. These sample vials are plugged with teflon coated butyl rubber closures and capped with dark blue, 13 mm flip off caps.

The potency, clarity and color data for Example 1 are reported in Table 2:

TABLE 2.

| POTENCY, CLARITY AND COLOR DATA FOR INJECTION COMPRISING TRIETHYLENETHIOPHOSPHORAMIDE IN POLYETHYLENE GLYCOL 300 | |
|---|---|
| Theoretical Potency: | 20 mg/ml |
| Label Potency: | 20 mg/ml |
| Proposed Potency: | 19.0-22.0 mg/ml (95-110% of label potency) |
| Proposed Clarity and Color: | clear, colorless to light yellow solution, free from turbidity. |

The mean assay yields for Example 1 are set forth in Table 3:

TABLE 3.

| MEAN ASSAY YIELDS FOR TRIETHYLENETHIOPHOSPHORAMIDE EXPRESSED AS PERCENT LABEL POTENCY WITH DESCRIPTION OF CLARITY AND COLOR | | | |
|---|---|---|---|
| Group No. | Storage Condition | % Label Potency | Description of Clarity and Color |
| 1 | 23° C - 0M (init.) | 95.0 | clear, colorless solution |
| 1 | 56° C - 2 WKS | 86.0 | clear, slightly yellow solution |
| 1 | 3° C - 13M | 98.5 | clear, colorless solution |
| 1 | 23° C - 13M | 83.0 | clear, colorless solution |
| 2 | 23° C - 0M (init.) | 103.5 | clear, colorless solution |
| 2 | 56° C - 2 WKS | 68.5 | clear, slightly yellow solution |
| 2 | 23° C - 13M | 90.5 | clear, colorless solution |
| WKS = weeks<br>M = months | | | |

A review of the stability data listed in Tables 2 and 3 for the Example 1 feasibility study indicates that the liquid pharmaceutical formulation for triethylenethiophosphoramide in polyethylene glycol 300 NF remains stable as a preconstituted solution for more than one year when stored under refrigerated conditions at 2° C to 8° C. The room temperature (23° C) and elevated temperature (56° C) samples showed a decided drop in potency as expected.

## EXAMPLES 2, 3, 4 AND 5

A second batch (Example 2) is prepared consisting of 3650 ml of composition. Triethylenethiophosphoramide is used as the active ingredient, and polyethylene 300 is used as the solvent,

in a composition set forth in Table 4:

TABLE 4.

| COMPOSITION FOR INJECTION COMPRISING TRIETHYLENETHIOPHOSPHORAMIDE | | |
|---|---|---|
| Ingredient | % W/V | Rationale |
| TRIETHYLENETHIOPHOSPHORAMIDE | 1.0 | active ingredient |
| Polyethylene Glycol 300 NF qs ad | 100.0 | vehicle |

A volume of polyethylene glycol 300 NF representing approximately 80% of the final batch volume is placed in the mixing vessel. Then 36.000 g of triethylenethiophosphoramide (98.8%) is weighed within a glove box into a pre-tared container and then wetted with 100 ml of polyethylene glycol 300 NF. All materials used in the weighing operation (spatulas, gloves, etc.) are collected in a toxic waste plastic bag, properly labeled. The triethylenethiophosphoramide-PEG 300 slurry mix is added to the PEG 300 (80% of the total volume) in the mixing vessel and mixed until dissolved. The batch is brought to final volume of 3,560 ml with PEG 300 and then processed through 0.2 micrometer sterile filtration in a Class 100 area. The sterile filtrate is filled into 5 ml, Type 1, clear glass vials at 1.6 ml per vial, then sealed with teflon coated 13 mm closures and aluminum crimp seals.

After the filling operation, the preparation, filtration and filling rooms and all involved equipment are treated with an antiseptic solution and hot water rinsings over a period of 5 days. Water rinse samples are collected on the 1st, 3rd and 5th days and are analyzed for residual triethylenethiophosphoramide by Quality Control. All exposed uniforms, gloves and goggles used by the operators are collected in toxic waste plastic bags, properly labeled.

A third batch (Example 3) consisting of 5,000 ml, a fourth batch (Example 4) consisting of 300 ml, and a fifth batch (Example 5) consisting of 300 ml of composition are prepared in the same manner as Example 2.

The potency, clarity and color data for Examples 2, 3, 4 and 5 are reported in Table 5.

TABLE 5.

| POTENCY, CLARITY AND COLOR DATA FOR INJECTION COMPRISING TRIETHYLENETHIOPHOSPHORAMIDE IN POLYETHYLENE GLYCOL 300 | |
|---|---|
| Theoretical Potency: | 10 mg/ml |
| Label Potency: | 10 mg/ml |
| Proposed Potency: | 9.5-11.0 mg/ml (95-110% of label potency) |
| Proposed Clarity and Color: | clear, colorless to light yellow solution, free from turbidity. |

The mean assay yields for Examples 2, 3, 4 and 5 are set forth in Table 6:

**TABLE 6. MEAN ASSAY YIELDS FOR TRIETHYLENETHIOPHOSPHORAMIDE EXPRESSED AS PERCENT LABEL POTENCY WITH DESCRIPTION OF CLARITY AND COLOR**

| Storage Condition | Example 2 % Label Potency | Example 3 % Label Potency | Example 4 % Label Potency | Example 5 % Label Potency |
|---|---|---|---|---|
| 23°C-0M (initial) | 99.0 | 103.5 | 97.7 | 103.0 |
| -10°C - 1M | 99.6 | 96.3 | 94.7 | - |
| -10°C - 12M | - | - | 100.6 | - |
| 3°C - 3M | 98.8 | - | - | - |
| 3°C - 6M | - | - | 100.6 | 103.0 |
| 3°C - 9M | 101.8 | - | 99.9 | 95.8 |
| 3°C - 12M | 102.6 | - | 100.6 | - |
| 23°C - 1M | - | 93.3 | - | 95.8 |
| 23°C - 2M | - | 98.5 | - | - |
| 23°C - 3M | 100.5 | - | - | - |
| 23°C - 12M | - | - | 60.3 | - |
| 37°C - 1M | - | 85.9 | - | 86.6 |
| 37°C - 2M | - | 78.1 | - | - |
| 37°C - 3M | 89.5 | - | - | - |
| 42°C - 2 WKS | - | 94.3 | - | 91.8 |
| 42°C - 1M | 99.0 | 70.7 | - | 82.6 |
| 42°C - 2M | - | 65.2 | 49.2 | - |

**TABLE 6 CONTINUED**

| Storage Condition | Example 2 Description | Example 3 Description | Example 4 Description | Example 5 Description |
|---|---|---|---|---|
| 23°C-0M (initial) | clear, colorless | clear, colorless | clear, colorless | clear, colorless |
| -10°C - 1M | clear, colorless | clear, colorless | clear, colorless | clear, colorless |
| -10°C - 12M | clear, colorless | clear, colorless | clear, colorless | - |
| 3°C - 3M | clear, colorless | clear, colorless | clear, colorless | - |
| 3°C - 6M | clear, colorless | clear, colorless | clear, colorless | clear, colorless |
| 3°C - 9M | clear, colorless | clear, colorless | clear, colorless | clear, colorless |
| 3°C - 12M | clear, colorless | clear, colorless | clear, colorless | clear, colorless |
| 3°C - 24M | clear, colorless | - | - | clear, colorless |
| 23°C - 1M | - | clear, colorless | - | clear, colorless |
| 23°C - 2M | - | clear, colorless | - | clear, colorless |
| 23°C - 3M | clear, colorless | clear, colorless | clear, colorless | - |
| 23°C - 12M | - | - | clear, colorless | - |

**TABLE 6 (CONTINUED)**

| Storage Condition | Example 2 Description | Example 3 Description | Example 4 Description | Example 5 Description |
|---|---|---|---|---|
| 37°C - 1M | - | clear, colorless | - | clear, colorless |
| 37°C - 2M | - | clear, slightly yellow-acceptable | - | clear, slightly yellow-acceptable |
| 37°C - 3M | clear, colorless | clear, slightly yellow-acceptable | slightly hazy slightly yellow-acceptable | - |
| 42°C - 2 WKS | - | clear, colorless | - | clear, colorless |
| 42°C - 1M | clear, colorless | clear, colorless | clear, colorless | clear, colorless |
| 42°C - 2M | - | clear, yellow unacceptable | clear, colorless | clear, slightly yellow acceptable |

The effectiveness of the preservative system of this invention for triethylenethiophosphoramide in polyethylene glycol 300, Example 2, is tested in accordance with the Antimicrobial Preservatives Effectiveness Test outlined in U.S. Pharmacopeia XX, pages 873-874. This provides a measure of the capacity of the solutions to decrease microbial growth when individually challenged with Staphylococcus aureus, Escherichia coli, Psuedomonas aeruginusa, Candida albicans and Aspergillus niger at a dose level of 100,000 to 1,000,000 microorganisms per ml. The contaminated solutions are stored and sampled at a series of time intervals in order to obtain microorganism counts. The result of this test, set forth in Table 7, indicates that the polyethylene glycol 300 prevents the growth of microorganisms, and provides qualities necessary to pass the anti-microbial preservative effectiveness test.

Table 7.

| Microbiological Evaluation of Preservative System - USP XX Method Example 2 | | | | | | | | |
|---|---|---|---|---|---|---|---|---|
| Storage Condition | Organism | Innoculum | 30 Min. | 24 Hours | 7 Days | 14 Days | 21 Days | 28 Days |
| 23° C - 0M | S. Aureus | 345,000 | 237,500 | 9250 | 100 | 10 | 10 | 10 |
| 23° C - 0M | E. Coli | 470,000 | 170,500 | 100 | 100 | 10 | 10 | 10 |
| 23° C - 0M | PS.Aeruginosa | 335,000 | 4200 | 100 | 100 | 10 | 10 | 10 |
| 23° C - 0M | C. Albicans | 570,000 | 111,500 | 3300 | 11,800 | 10 | 10 | 10 |
| 23° C - 0M | A. Niger | 265,000 | 27,000 | 26,500 | 2000 | 10 | 10 | 10 |

## EXAMPLE 6

Triethylenethiophosphoramide is used as the active ingredient, and dehydrated alcohol is used as the solvent, in this example.

A 1.0 percent w/v filtered solution of triethylenethiophosphoramide in dehydrated alcohol USP is filled at 1.8 ml into 2 ml glass ampuls. The ampuls are sealed and stored at 2° C to 8° C under refrigeration. The samples are assayed over the 4 month duration of the study and the results are given in Table 8.

TABLE 8.

| POTENCY, CLARITY AND COLOR DATA FOR TRIETHYLENETHIOPHOSPHORAMIDE IN DEHYDRATED ALCOHOL | | |
|---|---|---|
| Storage Condition | % Label Potency | Description of Clarity and Color |
| 2° C-8° C - Initial | 101.8 | clear, colorless solution |
| 2° C-8° C - 1M | 104.3 | clear, colorless solution |
| 2° C-8° C - 2M | 102.5 | clear, colorless solution |
| 2° C-8° C - 4M | 102.7 | clear, colorless solution |
| M = month(s) | | |

This product is projected to remain stable for at least one year when stored at 2-8° C. The dehydrated alcohol formulation, as with the polyethylene glycol 300 system, is self preserving. At time of use, the ampul contents are added to sufficient diluent, such as Water for Injection USP, to produce the desired therapeutic concentration, such as 1:60 (250 mcg/ml active) for the treatment of bladder carcinoma.

## EXAMPLE 7

If the procedure of Example 1 is repeated substituting triethylenemelamine for the triethylenethio phosphoramide as the active ingredient, using Polyethylene Glycol 300 NF qs ad as the vehicle, a stable pharmaceutical formulation in accordance with this invention will be obtained.

The foregoing data indicate that a unique blend of triethylenemelamine and the solvent endows the formulation with more stability than the presently available solution form reconstituted with water. More particularly, the formulation of this invention is shown to be a formulation which is more stable than the presently available solution form reconstituted with water in terms of ability to maintain potency under refrigerated conditions.

## EXAMPLE 8

If the procedure of Example 1 is repeated substituting triethylenephosphoramide for the triethylenethiophosphoramide as the active ingredient, using Polyethylene Glycol 300 NF qs ad as the vehicle, a stable pharmaceutical formulation in accordance with this invention will be obtained.

The foregoing data indicate that a unique blend of triethylenephosphoramide and the solvent endows the formulation with more stability than the presently available solution form reconstituted with water. More particularly, the formulation of this invention is shown to be a formulation which is more stable than the presently available solution form reconstituted with water in terms of ability to maintain potency under refrigerated conditions.

The above mentioned patents, publications and test methods are incorporated herein by reference.

The foregoing detailed description will suggest many obvious variations to those skilled in this art. For example, instead of antineoplastic compounds having three ethyleneimine groups, antineoplastic compounds having two ethyleneimine groups can be used, or a combination of antineoplastic compounds having more than one ethyleneimine group can be used. Instead of Polyethylene Glycol 300, Polyethylene 400 can be used. The solution can contain conventional additives such as chelating agents, inert gases, and the like. All such obvious modifications are within the full intended scope of the appended claims.

## Claims

1. A stable, injectable pharmaceutical composition, reconstituted with water from a solution comprising:
(i) an effective amount of an antineoplastic compound having more than one ethyleneimine group; and
(ii) a water-free, water-miscible, neutral or basic, oxygen containing pharmaceutically acceptable solvent therefor.

2. A composition as defined in Claim 1, wherein said antineoplastic compound
(i) comprises triethylenethiophosphoramide, triethylenemelamine, triethylenephosphoramide or a mixture of any of the foregoing:
(ii) comprises triethylenethiophosphoramide and
(iii) is selected from dehydrated alcohol, glycerine, a polyethylene glycol, propylene glycol, or a mixture of any of the foregoing.

3. A composition as defined in Claim 2, wherein said solvent
(ii) comprises a polyethylene glycol.

4. A stable, preconstituted pharmaceutical solution, adepted to form an injectable aqueous composition, said solution comprising:
(i) an effective amount of an antineoplastic compound having more than one ethyleneimine group; and
(ii) a polyethylene glycol.

5. A stable, preconstituted pharmaceutical solution, adepted to form an injectable aqueous composition, said solution comprising:
(i) from 5 mg/ml to 30 mg/ml of triethylenethiophosphoramide; and
(ii) polyethylene glycol 300.

6. A stable, preconstituted pharmaceutical solution, adapted to form an injectable aqueous composition, said solution comprising:
(i) from 9.5 mg/ml to 11.0 mg/ml of triethylenethiophosphoramide; and
(ii) polyethylene glycol 300.

7. A method for producing a stable, injectable pharmaceutical composition, said method comprising:
A. providing
(i) an effective amount of an antineoplastic compound having more than one ethyleneimine group;
B. adding thereto
(ii) a water-free, water-miscible, neutral or basic, oxygen containing pharmaceutically acceptable solvent; and
C. reconstituting the product of step B in water

8. A method fro producing a stable and preconstituted pharmaceutical solution, adapted to form an injectable aqueous composition, said method comprising:
A. providing
(i) an effective amount of an antineoplastic compound having more than one ethyleneimine group; and
B. adding thereto

(ii) a polyethylene glycol.

9. A method for producing a stable and preconstituted pharmaceutical solution, adapted to form an injectable aqueous composition, said method comprising:

A. providing

(i) triethylenethiophosphoramide; and

B. adding thereto

(ii) polyethylene glycol 300 in amount sufficient to provide said triethylenethiophosphoramide at a concentration of 5 mg/ml to 30 mg/ml.

10. A method for producing a stable and preconstituted pharmaceutical solution, adapted to form an injectable aqueous composition, said method comprising:

A. providing

(i) triethylenethiophosphoramide; and

B. adding thereto

(ii) polyethylene glycol 300 in amount sufficient to provide said triethylenethiophosphoramide at a concentration of 9.5 mg/ml to 11.0 mg/ml.